# EUROPEAN PATENT APPLICATION

(11) **EP 4 535 283 A1**
(43) Date of publication of application: **09.04.2025**
(21) Application number: 23210987.6
(22) Date of filing: 20.11.2023
(51) Int. Cl.: G06T 7/00, G06T 7/11

(54) **MEDICAL IMAGE PROCESSING**

(30) Priority: 03.10.2023 US 202363587521 P
(71) Applicant: Siemens Healthineers AG, 91301 Forchheim (DE)
(72) Inventor: ABDISHEKTAEI, Mohammad, Charlottesville, VA, 22903 (US); HERMOSILLO VALADEZ, Gerardo, West Chester, PA, 19382 (US)
(74) Representative: EIP

(57) **Abstract**

Medical image data (202) representing a medical image is received at a first ML system. The first ML system (204) generates, based on the received medical image data (202), a plurality of image embedding vectors (206) corresponding to a respective plurality of medical image features, each of the plurality of image embedding vectors relating to a different respective medical image feature and comprising medical image feature data indicative of the presence or absence of the respective medical image feature at each of a plurality of locations in the medical image. An indication (208) of a first medical image feature included in the medical image is received at a second ML system (210). The second ML system generates a feature vector (212) based on the indication. A comparison of the feature vector with the plurality of image embedding vectors is performed and a first image embedding vector is identified based on the comparison.

## Description

### Technical Field

The present invention relates to methods, apparatus and computer programs for segmenting medical image data.

### Background

Medical images can be segmented to identify regions of interest, such as organs or medical abnormalities. Segmentation can, among other things, allow quantitative data to be obtained from a medical image (e.g. the size of a certain medical feature), enable radiotherapy to be precisely planned, and enable the identification of features, such as medical abnormalities, that might not be noticed by a medical professional.

It is desirable to provide automated methods for processing medical images to identify such features and their locations in medical images, for purposes such as segmentation.

### Summary

According to a first aspect of the present invention, there is provided a computer implemented method for processing medical image data, the method comprising: receiving medical image data representing a medical image at a first machine learning-based system; the first machine learning-based system generating, based on the received medical image data, a plurality of image embedding vectors corresponding to a respective plurality of medical image features, each of the plurality of image embedding vectors relating to a different respective medical image feature and comprising medical image feature data indicative of the presence or absence of the respective medical image feature at each of a plurality of locations in the medical image; receiving, at a second machine learning-based system, an indication of a first medical image feature included in the medical image and generating, by the second machine learning-based system, a feature vector based on the indication; and performing a comparison of the feature vector with the plurality of image embedding vectors and identifying a first image embedding vector from among the plurality of image embedding vectors on the basis of the comparison.

Optionally, the first image embedding vector is identified as having a highest degree of similarity with the feature vector from among the plurality of image embedding vectors.

Optionally, the method comprises determining location data representing a location, in the medical image, of the indicated first medical image feature in the medical image data, based on the identified first image embedding vector.

Optionally, the medical image feature data represents a segmentation map of the respective medical image feature, and the determined location data comprises the segmentation map represented by the identified first image embedding vector.

Optionally, the segmentation map indicates probabilities that the respective medical image feature is present at respective locations in the medical image.

Optionally, the method comprises: determining whether the medical image represents a medical abnormality, and in response to determining that the medical image represents a medical abnormality, displaying the segmentation map on a display device.

Optionally, the indication received at the second machine learning-based system comprises area data representing an area of the first medical image feature in the medical image, and the comparison of the feature vector with the plurality of medical image embedding vectors is performed at least partly based on the area data.

Optionally, the indication received at the second machine learning-based system comprises data representing a text prompt indicating the first medical image feature.

Optionally, the method comprises performing a training process to train the first machine learning-based system and the second machine learning-based system, the training process comprising: inputting first training data comprising a plurality of sets of medical image data to the first machine learning-based system to generate, for each set of medical image data, a plurality of trial image embedding vectors; and inputting second training data to the second machine learning-based system to generate, for each set of medical image data, a trial feature vector, wherein the second training data comprises data representing a plurality of medical reports, each medical report comprising data indicating the presence, in a corresponding one of the sets of medical image data, of one of the plurality of medical image features, and each medical report comprises data representing an area, in the medical image represented by the corresponding set of medical image data, of the feature indicated as present, wherein the training process comprises jointly training the first machine learning-based system and the second machine learning-based system to minimize a loss function between the trial image embedding vectors and the corresponding trial feature vectors.

Optionally, the method comprises inputting each of the medical reports to a natural language processing system to generate a set of data representing findings of the medical report, wherein the data representing the plurality of medical reports is the data representing the findings of the medical reports.

Optionally, the method comprises inputting at least one of the plurality of image embedding vectors to a third machine learning-based system to generate natural language text describing a finding relating to the medical image data.

Optionally, the method comprises performing a training method to train the third machine learning-based system, the training method comprising: inputting image embedding vectors generated by the first machine learning-based system based on sets of input medical image data to the third machine learning-based system to generate, for each set of input medical image data, trial natural language text describing a finding relating to the set of input medical image data; and training the third machine learning-based system to minimize a loss function between the trial natural language text and data representative of medical reports corresponding to the sets of input medical imaging data.

Optionally, at least one of the plurality of medical image features comprises a medical abnormality.

According to a second aspect of the present invention, there is provided apparatus configured to perform the method according to the first aspect.

According to a third aspect of the present invention, there is provided a computer program which, when executed by a computer, causes the computer to perform the method according to the first aspect.

### Brief Description of the Drawings

Figure 1 is a flow diagram illustrating schematically a method for processing medical image data;
Figure 2 is a flow diagram illustrating schematically a specific example of the method illustrated in Figure 1;
Figure 3 is a flow diagram illustrating schematically an example method used by an image encoder 204 to generate a plurality of image embedding vectors;
Figure 4 is a flow diagram illustrating schematically a method for training machine learning-based systems to perform the method illustrated in Figure 1;
Figure 5 is a diagram illustrating schematically an apparatus according to an example.

### Detailed Description

Independent of the grammatical term usage, individuals with male, female or other gender identities are included within the term.

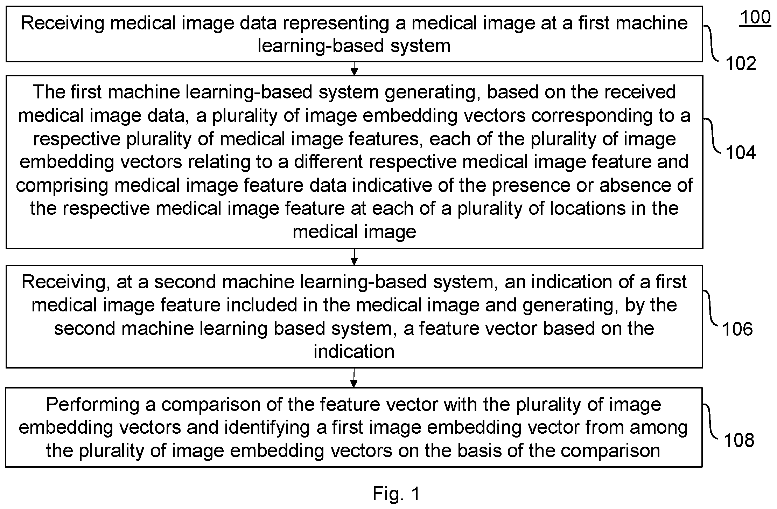

Figure 1 shows a flow diagram of a computer implemented method for processing medical image data 202.

In broad overview, the method comprises:
- in step 102, receiving medical image data 202 representing a medical image 202 at a first machine learning-based system;
- in step 104, the first machine learning-based system generating, based on the received medical image data 202, a plurality of image embedding vectors 206 corresponding to a respective plurality of medical image features, each of the plurality of image embedding vectors 206 relating to a different respective medical image feature and comprising medical image feature data indicative of the presence or absence of the respective medical image feature at each of a plurality of locations in the medical image 202;
- in step 106, receiving, at a second machine learning-based system, an indication of a first medical image feature included in the medical image 202 and generating, by the second machine learning based system, a feature vector 212 based on the indication; and
- in step 108, performing a comparison of the feature vector 212 with the plurality of image embedding vectors 206 and identifying a first image embedding vector from among the plurality of image embedding vectors 206 on the basis of the comparison.

The method thus enables medical image feature data indicative of the presence or absence of the respective medical image feature at each of a plurality of locations in the medical image, to be generated and identified. The comparison of the feature vector with the plurality of image embedding vectors allows an image embedding vector to be generated whose content relates to the medical image feature indicated at step 106.

The data indicating the presence or absence of the respective medical image feature at the locations, comprised by the identified image embedding vector, can be used in a variety of ways. As described below, a segmentation map indicating the probabilities that the indicated medical feature exists at each pixel in the medical image can be generated, and this can also be used by a medical professional for the purposes described in the background section. The identified image embedding vector can be used to determine whether the medical image represents a medical abnormality. Additionally, the identified image embedding vector can be used to generate text describing findings of the medical image. Furthermore, by generating image embedding vectors for a plurality of patients having respective medical abnormalities, and observing cluster patterns in the image embedding vectors, the respective medical abnormalities can be classified.

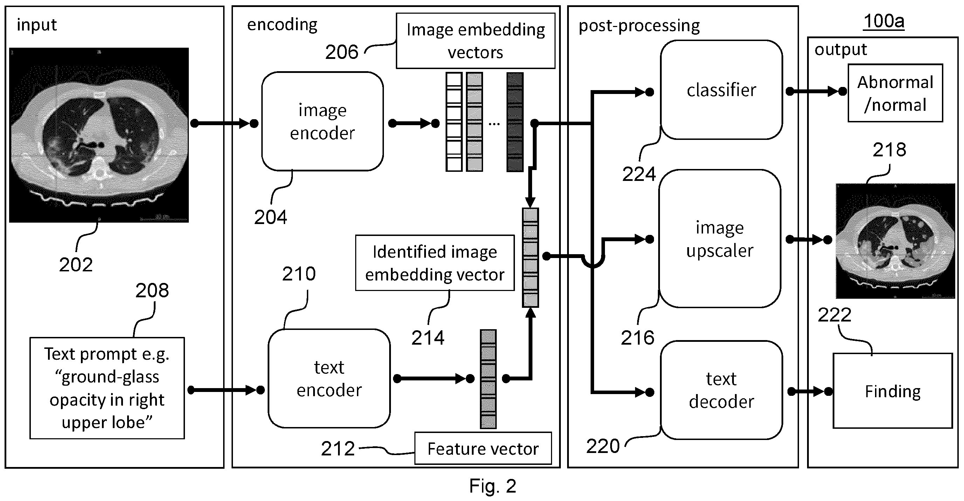

An example method 100a is now described in detail with reference to Figure 2. The method 100a of Figure 2 is a specific example of the method 100 of Figure 1 and includes the steps of the method 100 of Figure 1.

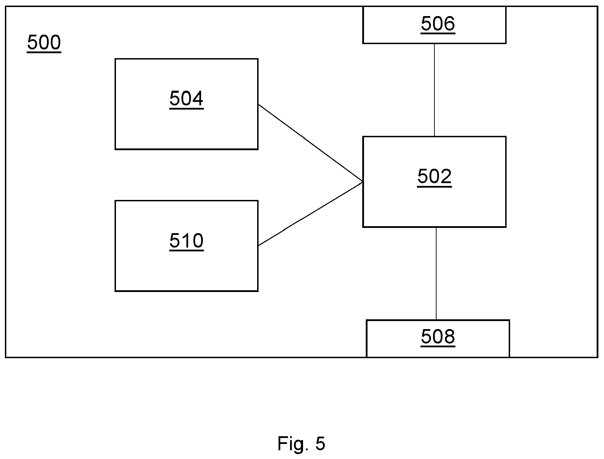

As mentioned, the method 100 comprises, in step 102, receiving medical image data 202 at a first machine learning-based system. In method 100a, the first machine learning-based system is an image encoder 204. Step 102 may involve retrieving the medical image data 202 from a storage such as a memory (see e.g. memory 504 in Figure 5, described in more detail below).

The medical image data 202 may comprise a 3D array of elements each having a value, which collectively represent a 3D medical image. The elements may be voxels, each voxel having at least one value. The at least one value may represent an output signal of the medical imaging technique used to generate the medical image data 202. For example, for Magnetic Resonance Imaging, the value of an element (e.g. voxel) may represent a rate at which excited nuclei, in a region corresponding to the element, return to an equilibrium state. As another example, in SPECT imaging, the value of an element may represent an amount of blood flow in capillaries represented by a given voxel. As another example, in CT imaging, the value of an element may correspond to or represent an amount of X-ray attenuation. In some examples, each element may only have one value. However, in other examples, each element may have or otherwise be associated with multiple values. For example, the multiple values of a given element may represent the values of respective multiple signal channels. For example, each signal channel may represent a different medical image signal or property of the imaging subject. In some examples, the at least one value may comprise an element (e.g. voxel) intensity value. For example, an output signal from the medical imaging may be mapped onto a voxel intensity value, for example a value within a defined range of intensity values. For example, for a greyscale image, the intensity value may correspond to a value in the range 0 to 255, where 0 represents a 'black' voxel and 255 represents a 'white' voxel, for example. As another example, for example as in the case of USHORT medical image data, the intensity value may correspond to a value in the range 0 to 65536. As another example, in a color image (e.g. where different colors represent different properties of the imaging subject) each pixel may have three intensity values, e.g. one each for Red, Green, and Blue channels. It will be appreciated that other values may be used. While the methods described herein chiefly use 3D medical images, it will be appreciated that they can be also applied to 2D medical images.

As mentioned, at step 104, the method 100 comprises the image encoder 204 generating, based on the received medical image data 202, a plurality of image embedding vectors 206 corresponding to a respective plurality of medical image features.

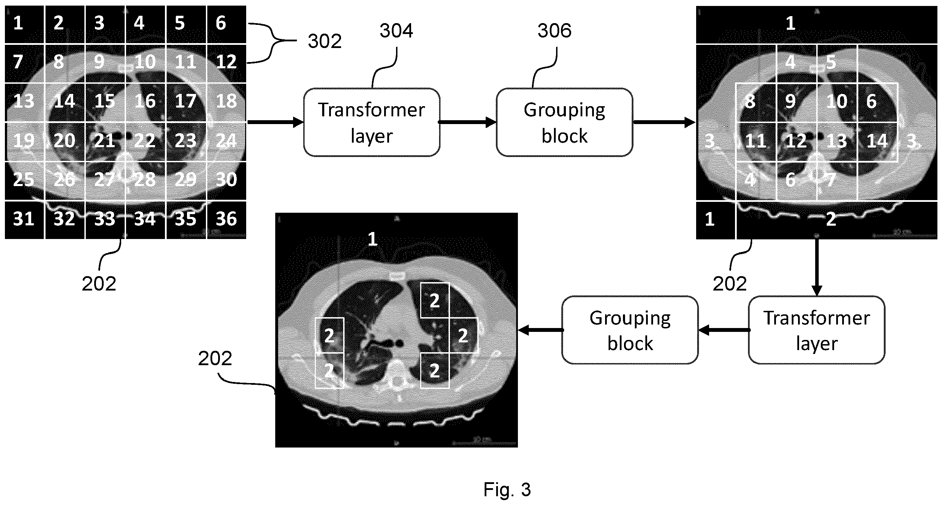

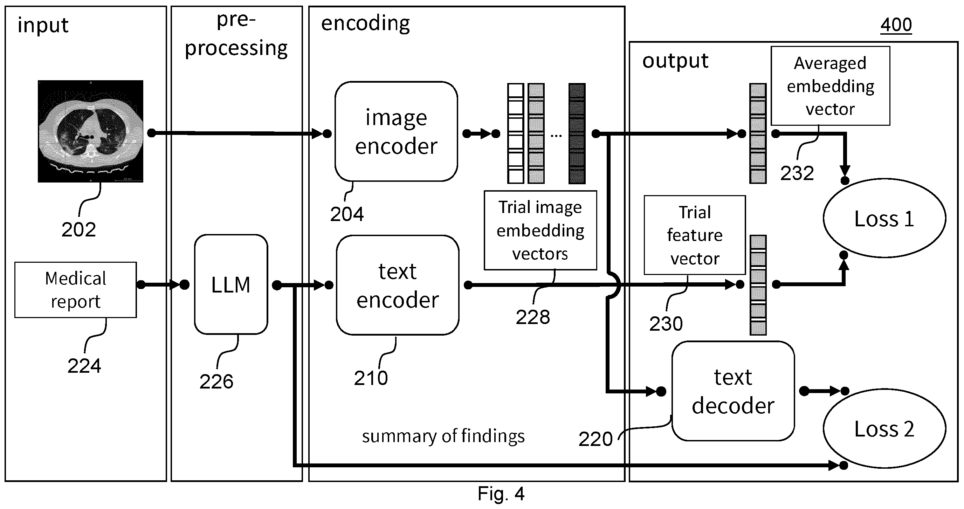

Method 100a comprises inputting the medical image data 202 to the image encoder 204 to generate the image embedding vectors 206. In method 100a, each of the plurality of medical image embedding vectors 206 relates to a different medical image feature. In method 100a, the medical image features are different medical abnormalities. The medical abnormalities in method 100a include ground-glass opacity and lesions. However, in some examples, the plurality of medical image embedding vectors 206 comprises two image embedding vectors, one relating to normal tissue and one relating to abnormal tissue. Furthermore, in some examples, the medical image features relate to different components of a body that are not medical abnormalities. For example, an image embedding vector may relate to a kidney, a pancreas, or a rib. The medical image features which the image embedding vectors 206 attend to depend on the contents of the training data used to train the image encoder 204, as described below with reference to Figure 4. An example method used by the image encoder to generate the image embedding vectors is described below with reference to Figure 3.

Each of the plurality of medical image embedding vectors 206 comprises medical image feature data indicative of the presence or absence of the respective medical image feature at each of a plurality of locations in the medical image 202. In method 100a, the feature data represents a segmentation map 218 of the medical image feature that the image embedding vector relates to, where the segmentation map 218 indicates probabilities that the respective medical image feature is present at respective locations in the medical image 202. In method 100a, each respective location is a portion 302 of the medical image 202. The first image embedding vector may represent a segmentation (probability) map for, for example, lesions. If there are no lesions in the medical image 202, the segmentation map 218 is likely to show a probability of 0 for every location in the image. The subject depicted in the medical image 202 in this example, however, has ground-glass opacity, and hence the segmentation map 218 of the image embedding vector for ground-glass opacity may show a non-zero probability for locations which have ground-glass opacity. In method 100a, the entries of a given medical image embedding vector are the above-mentioned probabilities, each component of the given medical image embedding vector corresponding to a different one of the image portions 302. However, in some examples, the medical image feature data is binary data indicating the presence or absence of the medical feature at each of the image portions. A "1" may indicate that the feature is present, while a "0" indicates that the feature is absent.

As mentioned, at step 106, the method 100 comprises receiving, at a second machine learning-based system, an indication of a first medical image feature included in the medical image 202 and generating, by the second machine learning based system, a feature vector 212 based on the indication.

In method 100a, the indication of the first medical image feature is a text prompt 208 indicating the first medical image feature. For example, a user such as a radiologist may input, through an input interface 506, to an apparatus 500 comprising the second machine learning-based system, text such as "ground-glass opacity". Alternatively, the user may select the first medical image feature from a drop-down box that lists the plurality of medical image features.

In method 100a, the indication includes area data representing an area, in the medical image 202, of the first medical image feature. For example, a radiologist may input, through the input interface 506, text such as "in right upper lobe". One text prompt 208 may include both the area and the first medical image feature; for example, the text prompt 208 may be "ground-glass opacity in upper right lobe". Alternatively, the area data may be inputted when a radiologist clicks on a certain area of a template image of a human body (or part thereof) stored by the apparatus 500 and displayed by a display device 510; for example, the apparatus 500 may store mappings between areas in the template image and names (such as "upper right lobe") for the respective areas, and retrieve the name of the area clicked on and use this as the text prompt 208.

In method 100a, the second machine learning-based system is a text encoder 210. The text encoder 210 generates, in this example, a feature vector 212 representing the text "ground-glass opacity in upper right lobe". The text encoder 210 may comprise a recurrent neural network which generates the feature vector 212. The feature vector 212 may have the same number of components as each of the image embedding vectors 206. The training of the text encoder 210 is described in detail below.

As mentioned, at step 108, the method 100 comprises performing a comparison of the feature vector 212 with the plurality of image embedding vectors 206 and identifying a first image embedding vector from among the plurality of image embedding vectors 206 on the basis of the comparison.

In method 100a, the apparatus 500 incorporating the text encoder 210 and the image encoder 204 generates, for each image embedding vector, a similarity measure indicating the degree of similarity between the image embedding vector and the feature vector 212. The identified (first) image embedding vector is chosen as the image embedding vector identified as having the highest similarity measure with the feature vector 212 of all of the image embedding vectors 206.

In method 100a, the image embedding vectors 206 and the feature vector 212 are represented in a common embedding space, and the similarity measure is a distance measure in the common embedding space between the image embedding vector and the feature vector 212. Any distance measure described herein may be, for example, a Euclidean distance or a cosine distance. By representing the image embedding vectors 206 and the feature vector 212 in a common embedding space, the distance measure can be used to identify the image embedding vector which is most closely aligned in its content with the feature vector 212. This enables an image embedding vector to be identified which represents a medical image feature that is described by the text prompt 208.

Since the feature vector 212 encodes information relating to the area of the medical image feature indicated at step 106, the comparison is based on the area data representing this area. The indication of the area in addition to the medical image feature provides further information on which to base the comparison, meaning that the image embedding vector identified through the comparison is more likely to pertain to the medical image feature which the medical professional desires to segment.

Method 100a comprises determining location data representing a location, in the medical image 202, of the medical image feature in the medical image data 202, based on the identified image embedding vector 214.

The location data can comprise the segmentation map 218 represented by the identified image embedding vector 214. The segmentation map 218 indicates probabilities that the medical image feature is present at respective locations in the medical image 202. The respective locations, in this example, are the locations of each pixel in the medical image 202.

In this example, the medical image feature data referred to in step 104 comprises the probabilities that the medical image feature is present at each of a plurality of locations in the medical image 202. As described above, each of these locations is a portion of the medical image 202. Each portion of the image comprises a plurality of voxels.

In this example, this medical image feature data is interpolated by an image upscaler 216 to provide, for each voxel of the medical image 202, a probability that the respective feature is present at the voxel. The interpolation may comprise, for example, trilinear interpolation. For example, the probability that the respective feature is present at a given voxel can be calculated by trilinear interpolation of the probabilities that the respective feature is present at each of the 8 portions of the image that are closest to the voxel. Thus, the low-dimension representation of the probability map can be upscaled to provide a full probability map for the medical image 202.

Indicating the probability of presence, rather than merely a binary indication of whether the feature is present or not, means that the segmentation map 218 can show blurred boundaries of a medical abnormality.

Method 100a comprises displaying the segmentation map 218 on a display device 510. The segmentation map 218 may be displayed on top of the medical image 202, so as to indicate the areas of the medical image 202 at which the abnormality is present. The display device 510 may be a computer monitor or other display screen of a computer and may be coupled to a processor 502 of the apparatus 500. A medical professional reviewing the segmentation map 218 displayed on the display device 510 may, for example, perform a qualitative evaluation of the extent of the abnormality using the segmentation map 218, and decide on a course of treatment on the basis of this evaluation.

The location data may additionally, or alternatively, comprise natural language text describing a finding 222 relating to the medical image data 202. Method 100a comprises inputting at least one, and optionally all, of the plurality of image embedding vectors 206 to a text decoder 220, also referred to as a fourth machine learning-based system, to generate this natural language text. The natural language text may comprise a finding 222 such as "There is ground-glass opacity in the upper lobe". The text decoder 220 may comprise a recurrent neural network, for example. The training of the text decoder 210 is described in further detail below with reference to Figure 4.

A medical professional may use the natural language text to, for example, diagnose a medical condition, or recommend treatment. In any case, by generating such natural language text, a radiologist or other medical professional can understand the condition of the patient, as represented by the image embedding vectors 206, by reading the natural language text.

Method 100a comprises performing a clustering process on a plurality of selected image embedding vectors determined from different sets of medical image data. For example, k-means clustering can be used to determine which of a plurality of clusters a particular image embedding vector belongs to. In this way, it can be determined whether two image embedding vectors, representing different sets of medical image data, represent the same medical abnormality.

Method 100a comprises determining whether or not the medical image 202 represents a medical abnormality, using a classifier 224. In one example, the classifier 224 is a large language model, for example ChatGPT, to which the generated natural language text is inputted, along with a text prompt such as "Does this represent a medical abnormality? Please answer yes or no.". In another example, the generated natural language text is inputted to a natural language processing system that compares each word in the natural language text describing the findings with a list of words that relate to medical abnormalities, such as lesion, and outputs an indication (e.g. a binary output) that the medical image 202 represents an abnormality if and only if the natural language text includes a word in the list.

In another example, the image embedding vectors 206 are all inputted to a fourth machine learning-based system to determine whether the medical image 202 represents a medical abnormality. The fourth machine learning-based system may be trained using supervised learning, for example by inputting image embedding vectors generated by the trained image encoder 204 to the fourth machine learning-based system to generate a trial classification of whether the medical image 202 represents a medical abnormality. The supervised learning comprises minimizing a loss function between the trial classifications and ground truth data representing whether the respective medical images used to generate the image embedding vectors represent a medical abnormality.

The output of the classifier 224 can be used to decide whether any further processing of the medical image 202 is required. In the case that the output of the classifier 224 indicates that the medical image 202 does not represent an abnormality, the medical image 202 can be removed from the radiology workflow. For example, the processor may display the segmentation map 218 on the display device 510, or perform other processing of the medical image 202, in response to determining that the medical image 202 represents a medical abnormality. In this example, if it is determined that the medical image 202 does not represent a medical abnormality, the processor 502 does not display the segmentation map 218 on the display device 510. The processor 502 may otherwise indicate on the display device 510 that the medical image 202 does not represent a medical abnormality, for example using text such as "No abnormality was detected in the image."

By determining whether the medical image 202 represents a medical abnormality and performing further processing in response to determining that the medical image 202 represents an abnormality, the efficiency of the analysis of the medical image 202 may be improved. Removing medical images that do not represent medical abnormalities from the radiology workflow reduces redundant processing of the medical images.

Figure 3 shows a flow diagram of an example process used by the image encoder 204 in method 100a to generate the image embedding vectors 206. The image encoder 204 is trained to generate, on the basis of the medical image data 202, the plurality of image embedding vectors 206. Its training process is described in detail below. The example image encoder 204 described below is a group vision transformer similar to that described in Section 3 of Xu et al., "GroupViT: Semantic Segmentation Emerges from Text Supervision", arXiv.org, 2022. However, other transformer-based attention mechanisms may be used, such as a Swin Transformer.

The image encoder 204 firstly divides the image into equal-sized portions 302, for example equal-sized rectangular portions 302. The image encoder 204 assigns, to each portion 302, a label. Initially, the labels are all different. In the illustrative 2D example shown in Figure 3, there are 6 x 6 = 36 portions 302 in total, labelled 1 to 36. In practice, the number of portions 302 would typically be much larger than this. Where the medical image data 202 is 3D, each portion 302 may comprise a cube of voxels in the medical image 202. For example, each portion 302 may include a cube of voxels with a side length of 3 voxels.

Each portion 302 is assigned, for each label, a probability that the portion 302 belongs to that label. The initially assigned probabilities simply represent that a portion 302 belongs to only one label and does not belong to any other label. For example, the top-left portion shown in Figure 3 has a 100% probability of belonging to label 1, and a 0% probability of belonging to any other label. The portion immediately to the right of this portion has a 100% probability of belonging to label 2, and a 0% probability of belonging to any other label.

The medical image data 202 of each portion 302 (e.g. voxels) is input to a transformer layer 304. The transformer layer 304 determines, for each pair of portions 302, a similarity metric. The similarity metric represents visual similarity between the portions 302.

The similarity metrics are input to a grouping layer 306. The grouping layer 306 groups together different portions 302 in accordance with the similarity metrics, to determine a number of labels that is smaller than the number of labels that existed prior to the grouping. In the example shown in Figure 3, the 36 initial labels are reduced to 14 intermediate labels after the first grouping layer 306. The numbers of labels before and after the grouping are fixed parameters of the image encoder 204.

The intermediate labels represent certain image features. For example, intermediate label 1 in the intermediate image of Figure 3 represents blank black space. Intermediate label 3 represents tissue exterior to the image subject's lungs. The other intermediate labels represent other image features.

By virtue of the transforming and grouping, each image portion 302 is associated with, for each intermediate label, a probability of belonging to that intermediate label. The intermediate labels shown in Figure 3 in each portion 302 of the medical image 202 represent the intermediate label that is associated with the highest probability for that portion. The black space at the top of the image is likely to have a probability close to 100% of belonging to intermediate label 1, and a probability close to 0% of belonging to any other intermediate label. Meanwhile, for example, the portion 302 which has a higher probability of belonging to intermediate label 10 than any other intermediate label, may have a probability of belonging to intermediate label 10 of 40%, a probability of belonging to intermediate label 9 of 30%, and a probability of belonging to intermediate label 8 of 10%. The probabilities for a given image portion do not need to add up to 100%.

The operations of transforming and grouping may be repeated a fixed number of times, until there is a desired number of labels. In the case shown in Figure 3, after the final stage of transforming and grouping, there are two final labels. Depending on the contents of the training data, the two final labels can represent certain medical image features; these form the plurality of medical image features referred to above. For example, final label 1 in Figure 3 represents normal tissue, while final label 2 represents abnormal tissue. Again, for simplicity, for each portion 302 of the medical image 202, only the label associated with the highest probability is shown. Each portion 302 has a probability of belonging to final label 1 (i.e., representing normal tissue), and a probability of belonging to final label 2 (i.e., representing abnormal tissue).

Each image embedding vector generated by the image encoder 204 is associated with one of the final labels, or medical image features. In this example, each component of the image embedding vector represents the probability that a respective image portion 302 belongs to the label, or medical image feature, associated with the image embedding vector.

Figure 4 shows a flow diagram of an example training process 400 for training the image encoder 204, text encoder 210 and text decoder 220 used in method 100a. In the example shown in Figure 4, the large language model (e.g. ChatGPT) is trained in a separate process, and is used in the training process 400 in its already trained form.

The training process 400 comprises inputting a plurality of sets of medical image data 202 to the image encoder 204 to generate, for each set of medical image data 202, a plurality of trial image embedding vectors 228. The trial image embedding vectors 228 referred to here are the image embedding vectors that are generated for the training process 400, as opposed to the image embedding vectors that are generated during the inference method 100 by the trained image encoder 204.

The sets of medical image data 202 may be selected from a bank of medical images. The selection process may involve selecting medical images that each contain one of the plurality of medical image features. For example, where the plurality of medical image features is a plurality of medical abnormalities, images that include one of these medical abnormalities may be selected, while images that include other abnormalities may be excluded. Where the plurality of medical image features comprises normal tissue and abnormal tissue, a number of sets of medical image data 202 depicting normal tissue may be selected, and an approximately equal number of sets of medical image data 202 depicting abnormal tissue may be selected.

The inventors have realized that, because medical reports typically include indications of medical image features along with associated images, they can be used as training data, with the indications in the reports acting as ground truth data. This avoids a difficulty with labeling medical image training data that a human implementer of the training process is typically unqualified to perform accurate assessment of medical images.. Thus, each set of medical image data 202 is associated with a medical report 224 for the medical image 202. The medical report 224 comprises data indicating the presence of one of the plurality of medical image features. For example, a medical report 224 may include text such as "There is a hypodense lesion". Each report also comprises data representing an area of the feature indicated as present. For example, a medical report 224 may include text such as "the right lobe of the liver". The report can be a report written by a medical professional or another person. The report may include the data indicating the presence of a feature in the same sentence as the data representing the area of the feature. For example, the medical report 224 may include a sentence such as "There is a hypodense lesion in the right lobe of the liver".

Through the first loss function described below, the area data can guide the image encoder 204 to attend to the parts of the image that include the medical image feature. For example, when an untrained image encoder 204 is only informed that there is a hypodense lesion somewhere in a medical image 202 used in the training, it is more likely to erroneously identify a part of the image that does not include the hypodense lesion, as including the hypodense lesion. Specifically, it might not output any image embedding vector which indicates a high probability for image portions 302 that include the hypodense lesion and a low probability for image portions 302 that do not include the hypodense lesion. Meanwhile, when an untrained image encoder 204 is informed that there is a hypodense lesion in the right lobe of the liver, it is more likely to pay attention to the correct part of the image. It is more likely to output at least one image embedding vector that correctly indicates high probability values for image portions 302 that include the hypodense lesion, and low probability values elsewhere. The inclusion of the area data may enable the image encoder 204 to be trained using fewer training examples, because the image encoder 204 can more easily identify parts of the image that it needs to attend. It may also improve the accuracy of the image embedding vectors produced by the trained image encoder 204, and hence improve the accuracy of the segmentation maps 218 described above.

In this example, the report is inputted to a natural language processing system to generate a set of data representing findings 222 of the medical report 224. The natural language processing system may be a large language model (LLM) 226, as shown in Figure 4, such as ChatGPT. Prior to input to the LLM 226, the medical report 224 may include information that is not useful for training the text encoder 210 and the image encoder 204. For example, the medical report 224 may include the patient's name, and metrics regarding the patient such as their height and weight. The medical report 224 may be inputted to the LLM 226 along with a prompt such as "Summarize the findings of this medical report 224". Data representing the output of the LLM 226 can then be inputted to the text encoder 210. This would exclude redundant information that may mislead the text encoder 210.

The text encoder 210 is configured to generate, based on the inputted summary of findings 222, a trial feature vector 230. The trial feature vector 230 effectively encodes information concerning the medical image feature (e.g. abnormality) described in the medical report 224, and the area in which the image feature is present.

The training process 400 comprises jointly training the image encoder 204 and the text encoder 210 to minimize a first loss function between the trial image embedding vectors 228 and the corresponding trial feature vectors 230. In this example, the trial image embedding vectors 228 generated for a given medical image are averaged to generate an averaged embedding vector 232. Each component of the averaged embedding vector 232 represents, for a respective image portion, the probability that the image portion 302 includes a given medical image feature, averaged over all medical image features. It should be noted that since the probabilities for a given image portion 302 need not add up to 1, the average of the probabilities is not necessarily constant, and is likely to vary across the image. It should also be noted that in this example, prior to the training, the image embedding vectors are not manually assigned to respective medical image features - this is instead enforced by the training, and the specific assignment of medical image features to image embedding vectors is not deliberately set by a user.

The first loss function measures the similarity between the generated trial feature vector 230 and the averaged image embedding vector 232. The similarity may be measured by Euclidean or cosine distances, for example. The value of the first loss function is higher the lower the level of similarity between the averaged image embedding vector 232 and the feature vector for a given medical image.

In some examples, a contrastive loss function may be used as the first loss function. For example, the medical images used in the training may be grouped into pairs, where each pair of medical images includes a normal medical image representing only normal tissue, and an abnormal medical image that represents a medical abnormality. The contrastive loss function additionally measures the similarity between the averaged image embedding vector 232 generated for the normal medical image and the averaged image embedding vector 232 generated for the abnormal medical image. The value of the contrastive loss function is higher the higher the level of similarity between the averaged image embedding vector 232 for the normal medical image and the averaged image embedding vector 232 for the abnormal medical image.

In any case, the first loss function can be used to update the parameters of the image encoder 204 and the text encoder 210. The weights of both the transformer layers 304 and the grouping layers 306 of the image encoder 204 can be updated. However, the number of labels before and after each grouping layer 306 is typically a fixed parameter of the image encoder 204. Furthermore, in this example, the parameters of the text decoder 220 are not updated using the first loss function.

Through the training, the image encoder 204 can be trained to generate, based on a medical image 202, image embedding vectors that represent the information that would be included in a summary of the findings 222 of a medical report 224 for the medical image 202.

The assignment of image embedding vectors to medical image features (e.g. different medical abnormalities) happens automatically and is enforced by the training. The medical report 224 for an abnormal medical image typically includes a finding that describes the abnormality. Thus, in order to minimize the first loss function, it is advantageous for the image encoder 204 to generate, for each medical image, at least one image embedding vector that represents a high probability value for image portions 302 that include the abnormality. Since the same abnormality is likely to be described in several medical reports 224 for respective medical images, generating such a vector reduces the total value of the first loss function for these medical images. Therefore, in order to minimize the first loss function, the image encoder will output image embedding vectors that describe appropriately abnormalities that are commonly referred to in the medical reports. The actual medical image features that are represented by the image embeddings therefore depend on firstly the contents of the medical reports, and secondly the number of image embeddings produced per medical image.

Hence, the image encoder 204 can be trained to generate, based on received medical image data 202, a plurality of image embedding vectors corresponding to a respective plurality of medical image features. In particular, the image encoder 204 can produce image embedding vectors, and hence segmentation maps, for each of a plurality of different types of abnormality.

Furthermore, using the first loss function, the text encoder can be trained to generate, based on an indication of a medical image feature, a feature vector based on the indication. The first loss function is minimized where the averaged image embedding vector 232 matches the trial feature vector 230 generated by the text encoder. Since a medical report describes a feature (e.g. abnormality) represented in the medical image 202, the text encoder will, in order to minimize the first loss function, generate a feature vector that represents the information encoded in the summary of findings generated by the LLM 226. If it does not do this and instead generates a feature vector at random, the image embedding vectors generated by the image encoder 204 will not match the feature vectors generated by the text encoder 210.

Some automatic segmentation methods can be used to identify well-defined medical image features. However, some features, including ground-glass opacity, and bones in X-rays, do not present well-defined boundaries when represented in a medical image. In other words, the transition between normal tissue and tissue affected by the medical image feature is gradual. Methods for segmenting images that involve determining a boundary between two segments of an image are thus inappropriate when segmenting such features.

To address this issue, a machine learning-based system may be trained using medical images for which a medical practitioner has manually indicated the boundary of an abnormality. However, this is very labor-intensive.

The above-described training process 400 and resulting runtime method 100a address this problem by using readily-available medical reports as training data.

As mentioned above, method 100a comprises inputting at least one of the image embedding vectors to a text decoder 220 to generate natural language text describing a finding 222 relating to the medical image data 202. We now describe an example training method for training the text decoder 220. The training method may form part of the training process 400.

The training method comprises inputting at least one image embedding vector generated by the image encoder 204 based on a set of input medical image data 202, to the text decoder 220. Based on the input image embedding vector, the text decoder 220 generates trial natural language text describing a finding 222 relating to the set of input medical image data 202.

The sets of training data used in the training using the first loss function can also be used to train the text decoder 220. As described above, these sets of training data include sets of medical image data 202 and corresponding medical reports 224. The text decoder 220 can be trained after the image encoder 204 and the text encoder 210 have been trained. Alternatively, the image encoder 204/text encoder 210 can be trained using the first loss function, and the text decoder 220 using the second loss function, in alternating training sessions. In any case, in some examples, during the training using the second loss function, the parameters of the image encoder 204 and the text encoder 210 are not updated.

In one example, the text decoder 220 generates the trial natural language text based on a plurality, or all, of the image embedding vectors generated by the image encoder 204 for a given set of input medical image data 202.

The training method comprises training the text decoder 220 to minimize a second loss function between the trial natural language text generated for each set of input medical image data 202, and data representative of the corresponding medical reports 224. In one example, the data representative of the medical reports 224 comprises the summaries of findings generated by the LLM 226 for the training using the first loss function. For example, a vector representing the natural language text generated by the LLM 226 can be compared with a vector representing the trial natural language text generated by the text encoder 210. Herein, natural language text can be represented by a vector by, for example, using a mapping between words in the natural language text and vectors such as word2vec, and taking an average of the vectors to produce a vector that represents the entirety of the natural language text. The comparison may involve calculating a Euclidean or cosine distance between the vectors, for example. On the basis of the comparison, the value of the second loss function for a given set of input medical image data 202 can be calculated. The value of the second loss function is higher the greater the distance between the vectors, i.e. the less the trial natural language text matches the summary of findings generated by the LLM 226.

The second loss function can be used to update the parameters (e.g. weights) of the text decoder 220. In this example, it is not used to update the parameters of the image encoder 204.

Through the training using the second loss function, the text decoder 220 can be trained to generate, based on at least one image embedding vector outputted by the image encoder 204 based on a medical image 202, natural language text that represents the information that would be included in a summary of findings of a medical report 224 for the medical image 202.

Figure 5 shows an apparatus 500 according to an example. The apparatus 500 may be implemented as a processing system and/or a computer. The apparatus 500 comprises an input interface 506, an output interface 508, a processor 502, a memory 504, and a display device 510. The processor 502 and the memory 504 may be configured to perform the method 100, 100a and/or the training process 400 according to any one of the examples described above with reference to Figures 1 to 4. The memory may store instructions which, when executed by the processor 502 cause the processor 502 to perform the method 100, 100a according to any one of the examples described above with reference to Figures 1 to 3, and/or the training method 400 according to any one of the examples described above with reference to Figure 4. The instructions may be stored on any computer readable medium, for example any non-transitory computer readable medium.

For example, the input interface 506 may receive medical image data 202, the processor 502 may implement the method 100a described above with reference to Figures 1 to 3 to identify an image embedding vector. The apparatus 500 may also display the segmentation map 218 of the identified image embedding vector 214 on the display device 510. In some examples, the natural language text generated by the text decoder 220 may be transmitted to a structured storage (not shown) so that the natural language text is stored in the structured storage.

The above examples are to be understood as illustrative examples of the invention. It is to be understood that any feature described in relation to any one example may be used alone, or in combination with other features described, and may also be used in combination with one or more features of any other of the examples, or any combination of any other of the examples. Furthermore, equivalents and modifications not described above may also be employed without departing from the scope of the invention, which is defined in the accompanying claims.

## Claims

1. A computer implemented method for processing medical image data, the method comprising:
receiving medical image data representing a medical image at a first machine learning-based system;
the first machine learning-based system generating, based on the received medical image data, a plurality of image embedding vectors corresponding to a respective plurality of medical image features, each of the plurality of image embedding vectors relating to a different respective medical image feature and comprising medical image feature data indicative of the presence or absence of the respective medical image feature at each of a plurality of locations in the medical image;
receiving, at a second machine learning-based system, an indication of a first medical image feature included in the medical image and generating, by the second machine learning-based system, a feature vector based on the indication; and
performing a comparison of the feature vector with the plurality of image embedding vectors and identifying a first image embedding vector from among the plurality of image embedding vectors on the basis of the comparison.

2. The method of claim 1, wherein the first image embedding vector is identified as having a highest degree of similarity with the feature vector from among the plurality of image embedding vectors.

3. The method of claim 1 or claim 2, comprising determining location data representing a location, in the medical image, of the indicated first medical image feature in the medical image data, based on the identified first image embedding vector.

4. The method of claim 3, wherein the medical image feature data represents a segmentation map of the respective medical image feature, and the determined location data comprises the segmentation map represented by the identified first image embedding vector.

5. The method of claim 4, wherein the segmentation map indicates probabilities that the respective medical image feature is present at respective locations in the medical image.

6. The method of claim 4 or claim 5, comprising:
determining whether the medical image represents a medical abnormality, and
in response to determining that the medical image represents a medical abnormality, displaying the segmentation map on a display device.

7. The method of any one of claim 1 to claim 6, wherein the indication received at the second machine learning-based system comprises area data representing an area of the first medical image feature in the medical image, and the comparison of the feature vector with the plurality of medical image embedding vectors is performed at least partly based on the area data.

8. The method of any one of claim 1 to claim 7, wherein the indication received at the second machine learning-based system comprises data representing a text prompt indicating the first medical image feature.

9. The method of any one of claim 1 to claim 8, comprising performing a training process to train the first machine learning-based system and the second machine learning-based system, the training process comprising:
inputting first training data comprising a plurality of sets of medical image data to the first machine learning-based system to generate, for each set of medical image data, a plurality of trial image embedding vectors; and
inputting second training data to the second machine learning-based system to generate, for each set of medical image data, a trial feature vector, wherein the second training data comprises data representing a plurality of medical reports, each medical report comprising data indicating the presence, in a corresponding one of the sets of medical image data, of one of the plurality of medical image features, and each medical report comprises data representing an area, in the medical image represented by the corresponding set of medical image data, of the feature indicated as present,
wherein the training process comprises jointly training the first machine learning-based system and the second machine learning-based system to minimize a loss function between the trial image embedding vectors and the corresponding trial feature vectors.

10. The method of claim 9, comprising inputting each of the medical reports to a natural language processing system to generate a set of data representing findings of the medical report, wherein the data representing the plurality of medical reports is the data representing the findings of the medical reports.

11. The method of any one of claim 1 to claim 10, comprising inputting at least one of the plurality of image embedding vectors to a third machine learning-based system to generate natural language text describing a finding relating to the medical image data.

12. The method of claim 11, comprising performing a training method to train the third machine learning-based system, the training method comprising:
inputting image embedding vectors generated by the first machine learning-based system based on sets of input medical image data to the third machine learning-based system to generate, for each set of input medical image data, trial natural language text describing a finding relating to the set of input medical image data; and
training the third machine learning-based system to minimize a loss function between the trial natural language text and data representative of medical reports corresponding to the sets of input medical imaging data.

13. The method of any one of claim 1 to claim 12, wherein at least one of the plurality of medical image features comprises a medical abnormality.

14. Apparatus configured to perform the method according to any one of claim 1 to claim 13.

15. A computer program which, when executed by a computer, causes the computer to perform the method according to any one of claim 1 to claim 13.
